# EUROPEAN PATENT APPLICATION

(11) **EP 3 616 693 A1**
(43) Date of publication of application: **04.03.2020**
(21) Application number: 18191289.0
(22) Date of filing: 28.08.2018
(51) Int. Cl.: A61K 31/185, A61K 31/19, A61K 31/191, A61K 9/00, A61K 9/06, A61K 47/38, A61K 33/04, A61P 31/12

(54) **THERAPY OF HIGH-RISK HUMAN PAPILLOMAVIRUS INFECTIONS**

(71) Applicant: Selo Medical GmbH, 5585 Unternberg (AT)
(72) Inventor: FUCHS, Norbert, 5580 Unternberg (AT); KUKLINSKI, Bodo, 18119 Rostock (DE)
(74) Representative: Sonn & Partner Patentanwälte

(57) **Abstract**

The present invention provides a pharmaceutical composition containing a selenite-containing compound and a pharmaceutically acceptable acid, selected from citric acid, acetic acid, malic acid, carbonic acid, sulfuric acid, nitric acid, hydrochloric acid, fruit acids and mixtures thereof. This composition is for use in the prevention or treatment of an infection of an internal reproductive organ of a female patient with at least one human papillomavirus (HPV) selected from HPV16, HPV18, HPV31, HPV33 and HPV58. The composition is applied intravaginally.

## Description

The present invention relates to lowering risk factors such as human papillomavirus (HPV), p16 and Ki-67 in the vaginal secretion as well as to prevention or treatment of a high-risk HPV infection of an internal reproductive organ such as the vagina or the cervix uteri.

Papillomaviruses are highly diverse (De Villiers, E. M., et al. (2004). Classification of papillomaviruses. Virology, 324(1), 17-27). There are more than 100 types of HPV, scattered within different genera and species of Papillomavirus, often bearing less than 55% sequence identity in their genome. Of these, only about 10%-15% are "high-risk" (i.e. a potential factor in oncogenesis), the others are benign, e.g. causing warts (verrucae).

The present standard of care against infection with high-risk HPV types is vaccination. For instance, Gardasil® (silgard) is a widely used recombinant HPV vaccine targeting HPV6, HPV11, HPV16 and HPV18, the latter of which are deemed to be high-risk. However, this vaccine is only suitable for prophylaxis and may hence be ineffective in older individuals who are more likely to have already been infected with one of these HPV types before vaccination. As for any systemic therapy, the risk of side effects of vaccinations tends to be higher than the risks of side effects in connection with local therapies. Furthermore, achieving vaccination compliance is challenging, as a portion of the general public is presently critical towards vaccination in general and HPV vaccines in particular.

It is hence an object of the present invention to provide an improved therapy of infection with a high-risk HPV such as HPV16 or HPV18, in particular a therapy which is effective in patients already having an infection with one of these HPV types and/or can be applied locally.

The present invention relates to a pharmaceutical composition containing a selenite-containing compound and a pharmaceutically acceptable acid, selected from citric acid, acetic acid, malic acid, carbonic acid, sulfuric acid, nitric acid, hydrochloric acid, fruit acids (e.g. malic acid, citric acid, tartaric acid, oxalic acid and fumaric acid, in particular citric acid) and mixtures thereof. The composition is for use in lowering risk factors such as HPV, p16 and/or Ki-67 in the vaginal secretion of a female patient, and/or for use in the (additional) prevention or treatment of an infection of an internal reproductive organ of a female patient with at least one HPV selected from HPV16, HPV18, HPV31, HPV33 and HPV58, in particular wherein the HPV is HPV16 and/or HPV18. The composition is applied intravaginally to the patient.

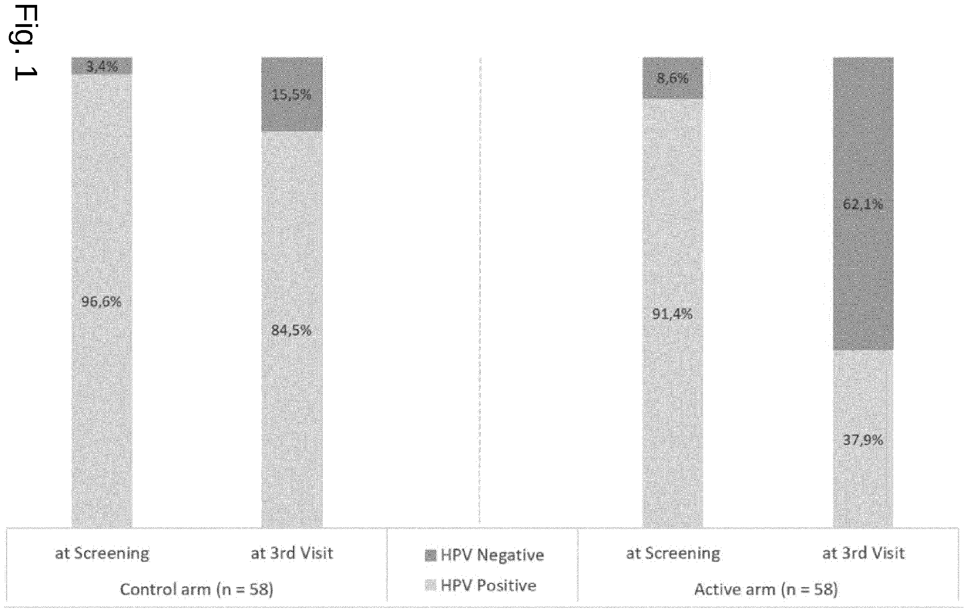

In the course of the present invention, it was surprisingly found that the pharmaceutical composition is effective against HPV16 as well as HPV18 when applied intravaginally, despite the fact that these two high-risk HPV types are evolutionarily very distant from each other (see de Villiers et al., Fig. 1). This was confirmed by a controlled clinical trial (see example 2).

While the pharmaceutical composition was previously disclosed for use against other therapeutic indications, these disclosures do not anticipate or suggest the present invention:

US 2003/0180387 A1 relates to a method for increasing the antioxidative potential of selenium-containing aqueous solutions. It is disclosed that a preparation comprising a pharmaceutically administrable or food-compatible form of selenium, namely selenite, and a pharmaceutically acceptable or food-compatible acid, selected from citric acid, acetic acid, malic acid, carbonic acid, various fruit acids and mixtures thereof, can be used to prevent or treat herpes simplex infections, among many other indications. However, the document neither discloses intravaginal application nor that the target organ is an internal reproductive organ of a female patient nor the use of a composition against papillomavirus.

US 2005/0048134 A1 concerns the use of a selenite-containing compound to be topically or buccally administered. Treatment or prophylaxis of infections with papilloma viruses, particularly in the genital region, is disclosed as one among many indications. However, the document neither discloses intravaginal application nor that the target organ is an internal reproductive organ of a female patient nor the use of a composition against the above-mentioned high-risk HPV types.

US 2013/0323328 A1 relates to a pharmaceutical preparation containing selenite or selenite-containing compounds for treating cervical dysplasia or carcinomas. However, the document is silent on prevention or treatment of infections in general and on the above-mentioned high-risk HPV types in particular. The document even teaches away from the present invention as it is stated that "the positive detection of an HPV infection often has no influence on subsequent therapeutic decisions" and that the disclosure "does not represent a strategy that is directed against a specific pathogen, but rather aims at a treatment of inflammations, dysplasias and/or carcinomas of the cervix in a directed manner, i.e. it may also be employed (long) after a potential pathogen has elicited the symptoms of a disease."

HPV infections may be specifically detected e.g. by polymerase chain reaction (PCR) or transcription-mediated amplification (TMA) assays of biopsy samples such as cervical smears, or other methods known in the art, since the genome sequences of HPV16, HPV18, HPV31, HPV33 and HPV58 are known. Genome sequences of these HPV types are for instance published in National Center for Biotechnology Information (NCBI) GenBank under the following accession numbers: K02718.1 (HPV16), X05015.1 (HPV18), J04353.1 (HPV31), M12732.1 (HPV33) and D90400.1 (HPV58).

p16 (also known as p16^{INK4a} or cyclin-dependent kinase inhibitor 2A) is a protein that is encoded by the *CDKN2A* gene in humans. Overexpression of p16 is a biomarker of certain transforming high-risk HPV infections associated with cancer. Ki-67 (also known as KI-67 or MKI67) is a protein that is encoded by the *MKI67* gene in humans. Ki-67 is a biomarker for cell proliferation. Both of these biomarkers may be tested for instance with the CINtec® PLUS test (Roche, Switzerland) which is a commercially available, approved cytological assay (see e.g. Ravarino, A., et al. (2012). CINtec PLUS immunocytochemistry as a tool for the cytologic diagnosis of glandular lesions of the cervix uteri. American Journal of Clinical Pathology, 138(5), 652-656) .

In the course of the present invention, it was found that the inventive therapy is especially effective against infections of HPV16, HPV18, HPV31, HPV33 or HPV58 when they are associated with biomarkers p16 and Ki-67. Accordingly, in a preferred embodiment of the present invention, the patient is p16-positive, preferably p16-positive and Ki-67-positive, at least in a region of the internal reproductive organ. Preferably, the pharmaceutical composition is applied until the patient has become p16-negative, preferably p16-negative and Ki-67-negative, in said region.

The skilled person knows how to assess whether a region of the internal reproductive organ is p16-positive, e.g. by performing a biopsy in the region and using a p16 assay (such as an immunocytochemistry assay) known in the art. Preferably, a biopsy sample obtained from the region of the internal reproductive organ is defined as p16-positive if at least one cell, preferably at least two adjoining cells, more preferably at least three adjoining cells, even more preferably at least five adjoining cells, in particular at least ten adjoining cells over-express p16 compared to healthy control tissue, such as healthy adjoining cells in the biopsy sample, preferably wherein each of said cells are epithelial cells. Conversely, such a biopsy sample is preferably defined as p16-negative if less than ten adjoining cells, preferably less than five adjoining cells, more preferably less than three adjoining cells, even more preferably less than two cells, in particular no cell over-expresses p16 compared to healthy control tissue, such as healthy adjoining cells in the biopsy sample, preferably wherein each of said cells are epithelial cells.

Further, the skilled person also knows how to assess whether a region of the internal reproductive organ is Ki-67-positive, e.g. by performing a biopsy in the region and using a Ki-67 assay (such as an immunocytochemistry assay) known in the art. Preferably, a biopsy sample obtained from the region of the internal reproductive organ is defined as Ki-67-positive if at least one cell, preferably at least two adjoining cells, more preferably at least three adjoining cells, even more preferably at least five adjoining cells, in particular at least ten adjoining cells over-express Ki-67 compared to healthy control tissue, such as healthy adjoining cells in the biopsy sample, preferably wherein each of said cells are epithelial cells. Conversely, such a biopsy sample is preferably defined as Ki-67-negative if less than ten adjoining cells, preferably less than five adjoining cells, more preferably less than three adjoining cells, even more preferably less than two cells, in particular no cell over-expresses Ki-67 compared to healthy control tissue, such as healthy adjoining cells in the biopsy sample, preferably wherein each of said cells are epithelial cells.

In a particular preference, "p16-positive and Ki-67-positive" is defined as a positive result in the established CINtec® PLUS test (Roche, Switzerland), especially as disclosed in CINtec® PLUS Interpretation Guide (Roche, 2016). In this Interpretation Guide, a positive result is defined as the presence of at least one dual-stained cervical epithelial cell (the cytoplasm stains brown (p16) and the nucleus stains red (Ki-67)).

In the course of the present invention, it turned out that the inventive composition is effective already at surprisingly low concentrations of selenium. Therefore, in a further preferred embodiment, the total selenium content of the pharmaceutical composition is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per 5 ml of the composition. It is evident that "total selenium content" does not imply that selenium has to be present as elemental selenium in the composition. By way of example, 0.83 mg sodium selenite as the only selenium-containing compound per 5 ml of composition corresponds to a total selenium content ("selenium-equivalent content") of 0.25 mg per 5 ml.

It was also found that the selenium dose per application can be low and still be effective. According to a further preferred embodiment, the total selenium dose is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per application. It is evident that "total selenium dose" does not imply that selenium has to be present as elemental selenium in the dose. By way of example, 0.83 mg sodium selenite as the only selenium-containing compound of a dosage unit applied corresponds to a total selenium dose ("selenium-equivalent dose") of 0.25 mg per application.

For increased effectiveness, it is preferred that the pharmaceutical composition is applied at least once per day, preferably for at least 30 days, more preferably for at least 60 days, even more preferably for at least 90 days.

It has turned out that application of the composition only once per day is suitable for achieving effectiveness. As this further reduces the risk of side effects compared to several applications per day, a further preferred embodiment relates to the inventive pharmaceutical composition for use wherein the composition is applied once per day, preferably for at least 30 days, more preferably for at least 60 days, even more preferably for at least 90 days.

The application of the pharmaceutical composition may be discontinued during menstruation of the patient.

According to a further preferred embodiment, the composition is applied until the infection of the internal reproductive organ or the region thereof cannot be detected any more.

The pharmaceutical composition for use according to the present invention may additionally contain further suitable ingredients and/or pharmaceutically acceptable excipients.

Preferably, the pharmaceutical composition for use according to the present invention contains selenite in the form of sodium selenite (which is mostly present as a pentahydrate compound which starts to release crystal water at 40°C).

In a further preferred embodiment of the present invention, the composition contains one or more acids in a total amount between 1 mg and 10 g acid, more preferably between 10 mg and 5 g acid, in particular between 100 mg and l g acid, per 100 g of the composition (in particular if the acid is added in its solid form). Alternatively, the acid may also be added in its liquid form (e. g. with water, i.e. as an aqueous solution). Water and aqueous solutions, respectively, optionally containing further ingredients, may be added to the composition according to the present invention in an amount between 0 and (about) 99.9 g, preferably between 50 and 99 g, in particular between 80 and 98g, per 100 g of the composition.

According to a further preferred embodiment, the composition is present in the form of a gel, a suspension, an emulsion, a suppository such as gelatine capsules or gelatine-free capsules, a spray or a powder.

When present in the form of a gel, the pharmaceutical composition for use according to the present invention preferably contains a gelling agent. Both inorganic and organic aqueous gelling agents may be used as a gelling agent. Particularly suitable gelling agents are cellulose derivatives, in particular carboxymethylcellulose, methylcellulose, hydroxypropyl cellulose and, in particular, hydroxyethyl cellulose. Preferably, the gelling agents, in particular hydroxyethyl cellulose, are used at a total concentration of between 0.1 g and 30 g, more preferably between 0.5 g and 5 g, in particular between 1 g and 3 g, per 100 g of the composition.

A further particularly preferred embodiment of the composition for use according to the present invention, especially when the composition is present in the form of a gel, contains silicon dioxide, in particular highly dispersed silicon dioxide, e. g. according to WO 2001/85852 A1, as a technological suspension medium and/or as an adsorbent. Preferably, an amount between 100 mg and 50 g, more preferably between 500 mg and 10 g, in particular between 1 g and 5 g, SiO2 per 100 g of the composition is used.

The composition for use according to the present invention preferably has a pH-value of less than 7.0, more preferably less than 5.0, in particular between 4.0 and 2.5.

Advantageously, the composition may contain further excipients and/or further active ingredients, in particular buffer substances, colouring agents, stabilizers, preservatives, carrier substances or combinations thereof. Preferred examples of preservatives are potassium sorbate and sodium benzoate.

Moreover, the composition may additionally comprise further active agents such as antibiotics, antiviral agents, antimycotics, pain inhibitors, anti-inflammatory agents or combinations thereof.

The present invention also relates to a method for delaying the onset of or treating an infection of an internal reproductive organ of a female patient with at least one HPV selected from HPV16, HPV18, HPV31, HPV33 and HPV58, comprising
- obtaining a pharmaceutical composition as defined herein; and
- administering an effective amount of the composition to the patient, wherein the composition is applied intravaginally.

In the entire context of the present invention, the term "preventing" or "prevention" as used herein means to stop a disease state or condition from occurring in a patient or subject completely or almost completely or at least to a (preferably significant) extent, especially when the patient or subject is predisposed to such a risk of contracting a disease state or condition.

Typically, the patient (who preferably has an infection of an internal reproductive organ with at least one HPV selected from HPV16, HPV18, HPV31, HPV33 and HPV58 (in particular HPV16 and/or HPV18), and especially has been diagnosed with said infection) is in need of the inventive treatment. In the context of the inventive treatment for prevention of the HPV infection, the patient may be a patient that does not have the HPV infection but is at risk of developing the HPV infection.

Herein, the patient to be subject to inventive prevention or treatment is preferably older than 20 years, preferably older than 30 years, even more preferably older than 40 years, yet even more preferably older than 50 years.

The inventive treatment has been found to be not as efficient in immunosuppressed patients. Therefore, the patient is preferably not immunosuppressed. In addition, or alternatively thereto, the patient preferably does not have cancer and/or chronic viral disease (other than HPV infection).

Preferably, the internal reproductive organ mentioned herein is the vagina or the cervix uteri of the patient.

Herein, the term "over-express" or similar in regard to a gene (product) A typically can mean that the expression level of A (as e.g. measured by Western blot or immunohistochemistry or immunocytochemistry) e.g. in a biopsy sample is higher than the expression level of an appropriate control (such as healthy tissue of the same type), by a factor of at least 1.2 (i.e. an increase of at least 20%), preferably at least 1.4, more preferably at least 1.6, even more preferably at least 1.8, especially at least 2.0.

The present invention is further illustrated by the following figures and examples, without being restricted thereto.

**Fig. 1****: Clearance of HPV types 16, 18, 31, 33 and 58.** Compared to the non-treated control arm a much larger clearance rate of HPV16, HPV18, HPV31, HPV33 and HPV58 was observed in the active arm treated with the inventive therapy. "HPV Negative": no presence of HPV16, HPV18, HPV31, HPV33 and HPV58 detected. "HPV Positive": presence of HPV16, HPV18, HPV31, HPV33 and/or HPV58 detected. "at Screening": initial screening of the patient, "at 3^{rd} Visit": after 3x28 days of once-daily treatment.

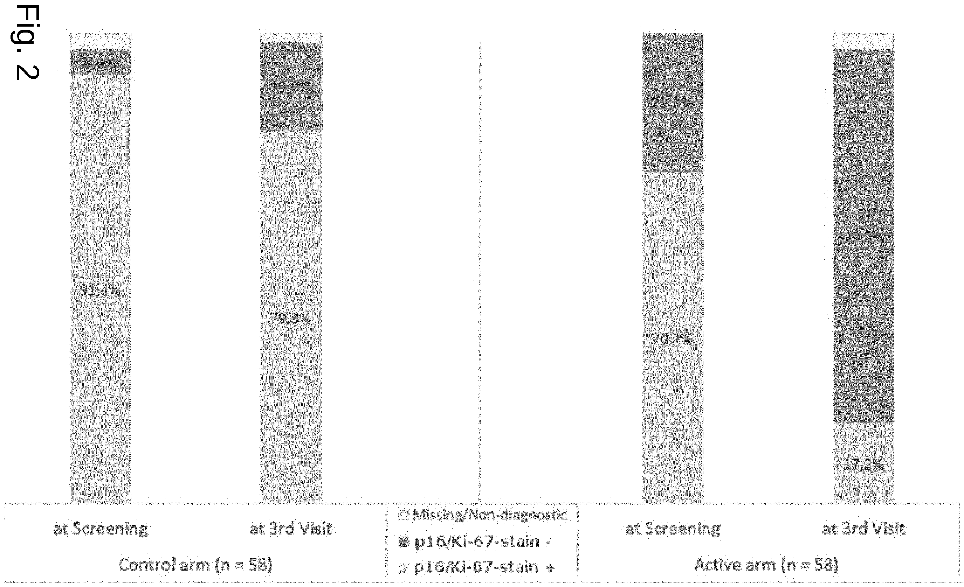

**Fig. 2****: Improvement of p16/Ki-67 status.** Compared to the non-treated control arm, a much larger remission rate from p16/Ki-67 double-stain positive was observed in the active arm treated with the inventive therapy. "p16/Ki-67-stain -": negative as determined with the established CINtec® PLUS test (Roche, Switzerland). "p16/Ki-67-stain +": positive as determined with the established CINtec® PLUS test (Roche, Switzerland). "at Screening": initial screening of the patient, "at 3^{rd} Visit": after 3x28 days of once-daily treatment.

### Example 1 - Pharmaceutical composition for intravaginal therapy of infection with HPV types 16, 18, 31, 33 and 58

The composition is an aqueous vaginal gel having the following ingredients (per 5ml of gel):

| | |
|---|---|
| highly dispersed silicon dioxide | 10.00 mg |
| citric acid | 24.80 mg |
| sodium selenite (corresponds to 0.25 mg selenium equivalent) | 0.83 mg |
| sodium benzoate | 2.50 mg |
| potassium sorbate | 5.00 mg |
| hydroxyethyl cellulose | 100.00 mg |
| water | (up to 5 ml) |

### Example 2 - Controlled clinical trial

The present trial was a randomized, prospective, open label with control group multicentre study of 3 x 28 days once a day treatment with the composition of example 1 (n = 116 female patients, age 25-60 years, no chronic viral disease, cancer and immunosuppressive treatment). Daily dose was 5 ml of the composition, applied intravaginally.

58 patients were in the active arm (treated with the composition of example 1) and 58 in the control arm (non-treated). Mean age in active arm was 34.5 +/- 7.4 years, and 33.6 +/- 7.5 years in control arm.

The subjects were recruited in 3 centres. Cervical smears were collected according the standard procedure known in the art, preserved in a specific liquid milieu called SurePath® (Becton & Dickinson). Samples were analysed with, inter alia, the CINtec® PLUS cytology kit (Roche, Switzerland).

The clinical trial was conducted in accordance with ethical principles of the Declaration of Helsinki 2013 and ISO 14155: 2011 + Cor 1: 2011.

### Results

Evaluated data demonstrate a clearly improved outcome for patients in the active arm for all measured parameters. Only a few slight adverse events were reported.

Compared to the non-treated control arm a much larger clearance rate of HPV16, HPV18, HPV31, HPV33 and HPV58 was observed in the active arm treated with the inventive therapy (see Fig. 1).

Further, compared to the non-treated control arm, a much larger remission rate from p16/Ki-67 double-stain positive was observed in the active arm treated with the inventive therapy (see Fig. 2).

These results demonstrate the exceptional suitability of the composition for the HPV indications mentioned herein.

## Claims

1. A pharmaceutical composition containing a selenite-containing compound and a pharmaceutically acceptable acid, selected from citric acid, acetic acid, malic acid, carbonic acid, sulfuric acid, nitric acid, hydrochloric acid, fruit acids and mixtures thereof, for use in the prevention or treatment of an infection of an internal reproductive organ of a female patient with at least one human papillomavirus (HPV) selected from HPV16, HPV18, HPV31, HPV33 and HPV58, wherein the composition is applied intravaginally.

2. The pharmaceutical composition for use according to claim 1, wherein the patient is p16-positive, preferably p16-positive and Ki-67-positive, at least in a region of the internal reproductive organ, preferably wherein the composition is applied until the patient has become p16-negative, preferably p16-negative and Ki-67-negative, in said region.

3. The pharmaceutical composition for use according to claim 1 or 2, wherein the total selenium content is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per 5 ml of the composition.

4. The pharmaceutical composition for use according to any one of claims 1 to 3, wherein the total selenium dose is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per application.

5. The pharmaceutical composition for use according to any one of claims 1 to 4, wherein the composition is applied at least once per day, preferably for at least 30 days, more preferably for at least 60 days, even more preferably for at least 90 days; optionally wherein the application is discontinued during menstruation of the patient.

6. The pharmaceutical composition for use according to any one of claims 1 to 5, wherein the composition is applied once per day, optionally wherein the application is discontinued during menstruation of the patient.

7. The pharmaceutical composition for use according to any one of claims 1 to 6, wherein the application is discontinued during menstruation of the patient.

8. The pharmaceutical composition for use according to any one of claims 1 to 7, wherein the composition is applied until the infection of the internal reproductive organ cannot be detected any more.

9. The pharmaceutical composition for use according to any one of claims 1 to 8, wherein the composition is present in the form of a gel, a suspension, an emulsion, a suppository such as gelatine capsules or gelatine-free capsules, a spray or a powder.

10. The pharmaceutical composition for use according to any one of claims 1 to 9, wherein the composition further contains silicon dioxide, preferably highly disperse silicon dioxide.

11. The pharmaceutical composition for use according to any one of claims 1 to 10, wherein the composition has a pH of less than 7.0, preferably less than 5.0, in particular between 4.0 and 2.5.

12. The pharmaceutical composition for use according to any one of claims 1 to 11, wherein the pharmaceutically acceptable acid is citric acid.

13. The pharmaceutical composition for use according to any one of claims 1 to 12, wherein the selenite-containing compound is sodium selenite.

14. The pharmaceutical composition for use according to any one of claims 1 to 13, wherein the internal reproductive organ is the vagina or the cervix uteri of the patient.

15. The pharmaceutical composition for use according to any one of claims 1 to 14, wherein the patient does not have cancer and/or chronic viral disease other than HPV infection, and/or wherein the patient is not immunosuppressed.
